(19) Europäisches Patentamt
European Patent Office
Office européen des brevets

(11) Publication number: **0 186 023**
**A2**

(12) # EUROPEAN PATENT APPLICATION

(21) Application number: 85115611.7

(22) Date of filing: 08.12.85

(51) Int. Cl.[4]: C 07 J 9/00
A 61 K 31/575

(30) Priority: 21.12.84 IT 2418784

(43) Date of publication of application:
02.07.86 Bulletin 86/27

(84) Designated Contracting States:
AT BE CH DE FR GB IT LI LU NL SE

(71) Applicant: LEHNER A.G.
Birsfelderstrasse 44
CH-4132 Muttenz(CH)

(72) Inventor: Castagnola, Virginio
Via Palagi, 2
I-20100 Milan(IT)

(72) Inventor: Frigerio, Giuliano E.
Via Palagi, 2
I-20100 Milan(IT)

(74) Representative: Braun, André et al,
A. Braun, Braun, Héritier, Eschmann AG Patentanwälte
Holbeinstrasse 36-38
CH-4051 Basel(CH)

(54) **Biliary acid derivatives, process for their preparation and pharmaceutical compositions containing them.**

(57) Compounds of general formula (I)

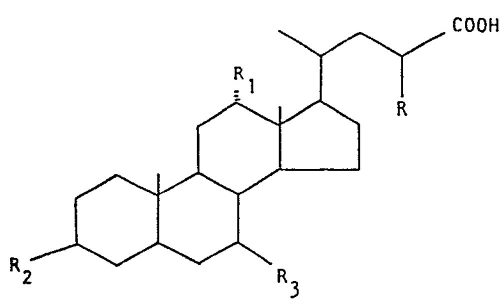

(I)

wherein:

R is hydrogen, methyl or ethyl;

$R_1$ is a $C_1$-$C_3$ alkoxy group and, when R is ethyl, $R_1$ may also be hydrogen or hydroxy;

$R_2$ and $R_3$, which may be the same or different, represent hydroxy groups both of α and β configuration, or keto groups.

Compounds I are useful in therapy.

EP 0 186 023 A2

# BILIARY ACID DERIVATIVES, PROCESS FOR THEIR PREPARATION AND PHARMACEUTICAL COMPOSITIONS CONTAINING THEM

The present invention relates to biliary acids, a process for the preparation thereof and pharmaceutical compositions containing them. Particularly, the invention relates to compounds of formula I

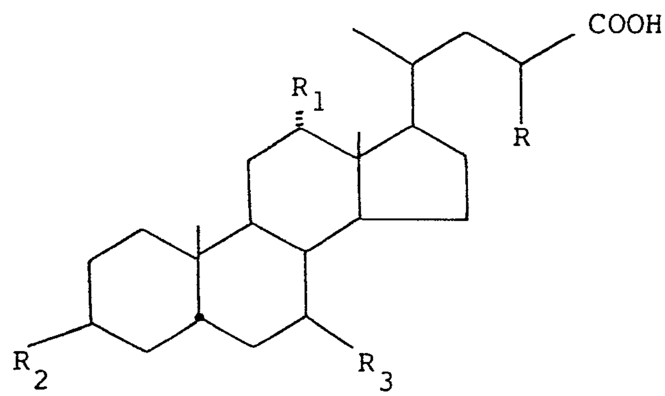

(I)

wherein:

R is hydrogen, methyl or ethyl;

$R_1$ is a $C_1$-$C_3$ alkoxy group and, when R is ethyl, $R_1$ may also be hydrogen or hydroxy;

$R_2$ and $R_3$, which may be the same or different, represent hydroxy groups both of α and β configuration, or keto groups.

Compounds of formula I have a number of asymmetric carbon atoms, therefore the invention relates also to all the possible isomers, both separated and in racemate or diastereoisomeric forms.

The invention further relates to pharmaceutically acceptable inorganic and organic base addition salts, such

as sodium, potassium, calcium, magnesium, triethylamine, lysine, arginine, diethanolamine ecc. salts, and also conjugated derivatives with taurine and glycine.

Compounds I exhibit marked pharmacological activities, particularly choleretic activity, and are therefore used in human therapy, for example as choleretics, eupeptics, antidyspeptics, andidyslipidemics, in the treatment of calculosis and generally in all those pathologic conditions wherein a stimulus of the bile flow and a qualitative and quantitative modification of the biliary composition are necessary.

An essential aspect of the present invention is therefore provided by pharmaceutical compositions containing as active principle at least one of the compounds of formula I, or salts or conjugated thereof.

A preferred class of compound according to the invention is represented by compounds of formula I wherein R is methyl and $R_1$ is methoxyl.

Another preferred class of compounds is that in which R is hydrogen and $R_1$ is methoxy or that in which R is ethyl and $R_1$ is hydrogen, hydroxy or methoxy.

Examples of preferred compounds are:

- 3α-7β-dihydroxy-12α-methoxy-23-methyl-5β-cholanoic acid
- 3α-7β-dihydroxy-12α-methoxy-23-ethyl-5β-cholanoic acid
- 3α-7α-dihydroxy-12α-methoxy-23-methyl-5β-cholanoic acid
- 3α,7α-dihydroxy-12α-methoxy-5β-cholanoic acid
- 3α,7α-dihydroxy-23-ethyl-5β-cholanoic acid
- 3α,7β-dihydroxy-23-ethyl-5β-cholanoic acid
- 3α,7β-dihydroxy-12α-methoxy-5β-cholanoic acid
- 3α,7α,12α-trihydroxy-23-ethyl-5β-cholanoic acid.

Compounds I may be prepared from natural biliary acids of formula II

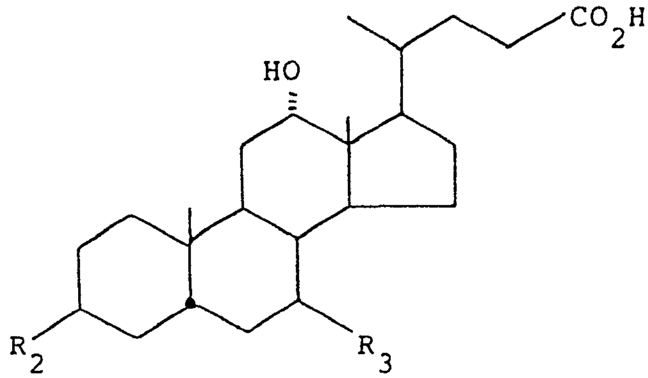

(II)

wherein $R_2$ and $R_3$ have the above mentioned meanings.

Compounds II, after protection of the hydroxyl groups at the 3- and 7-positions, as well as the free carboxylic group, are reacted with lithium-alkyls, in the presence of anhydrous aprotic solvents and subsequently with alkyl halides having 1 to 3 carbon atoms.

Finally, compounds of formula I are obtained by removing the protecting groups and hydrolyzing the ester used to protect the carboxy group. According to the process of the invention, it is therefore possible to have alkylation in position $\alpha$ of the carboxy group or alkylation of the hydroxy group in position $12\alpha$.

It is possible to choose the C-alkylation or the O-alkylation using techniques well known to the skilled in the art, for example suitably selecting the solvents and the reaction conditions.

The reaction does not substantially proceed in case of alkyl halides having more than 3 carbon atoms, due to steric hindrance, the introduction of an ethyl group in

the α configuration - which yields a series of new compounds, even without the presence at the same time of the 12α methoxy group - requiring more drastic conditions.

Finally, the conjugated of compounds I may be obtained starting from the corresponding biliary acids, by reaction with taurine or glycine, in the presence of EEDQ (N-ethoxycarbonyl-2-ethoxy-1,2-dihydroquinoline), according to the disclosure of our italian patent application No. 22572 A/83, filed on August 18th, 1983.

The following examples further illustrate the present invention, without limiting it in no way.

EXAMPLE 1

a) Methyl 3α,7ß-dicathyl-12α-hydroxy-5ß-cholanoate

3.7 Ml of ethyl chlorocarbonate were added dropwise to a solution of ursocholic acid methyl ester (5.5 g) in anhydrous dioxane (400 ml) and pyridine (8 ml) at 0°C, under magnetic stirring.

The reaction mixture was stirred at room temperature for 6 hours, then it was poured into water. The resulting solution was extracted with some portions of ethyl ether, and the combined organic extracts were washed with a 10% HCl solution, then with a saturated NaCl solution, dried over anhydrous $MgSO_4$ and evaporated under vacuum. The residue was purified by chromatography on silica gel, eluting with petroleum ether - ethyl ether. 5.5 Grams of the dicathylate derivative were obtained.

b) Methyl 3α,7ß-dicathyl-12α-methoxy-23-methyl-5ß-cholanoate

11 Ml of n-butyl-lithium were added to a solution of N-cyclohexyl-N-isopropylamine (2.8 ml) in anhydrous

THF, and the mixture was cooled to -78°C. 2.5 Grams of the dicathylderivative methylester prepared in step a), and subsequently 10.2 g of methyl iodide, were added.

The reaction mixture was stirred for 5 hours, then acidified with a 10% HCl solution and extracted with some portions of chloroform. The combined organic fractions were washed with water, dried over anhydrous $Na_2SO_4$ and evaporated under vacuum.

By silica gel chromatography, eluting with petroleum ether/ethyl ether, a pure fraction was obtained.

I.R. ($CHCl_3$) = 1737 $cm^{-1}$

$^1$H-NMR ($CDCl_3$) $\delta$ = 0.67 (d, 3H, C-18, Me); 0.77 (s, 3H, C-19, Me); 1.10 (t, 6H, $OCH_2\underline{CH_3}$); 3.20 (s, 3H, C-12, OMe); 3.37 (m, 1H, C-12, C<u>H</u>OMe); 3.60 (s, 3H, COO<u>Me</u>); 4.16 (g, 6H, $O\underline{CH_2}CH_3$); 4.53 (m, 2H, C-3 and C-7, <u>CH</u>$OCO_2$Et).

c) <u>3α,7β-Dihydroxy-12α-methoxy-23-methyl-5β-cholanoic acid</u>

Methyl 3α,7β-dicathyl-12α-methoxy-23-methyl-5β-cholanoate (3) (600 mg) was treated under reflux overnight with an hydroalcoholic solution of 15% NaOH. Thereafter the reaction mixture was poured into water, acidified with concentrated HCl, extracted with some portions of chloroform; the combined organic fractions were washed with a saturated NaCl solution and dried over anhydrous sodium sulfate. The resulting residue was purified by chromatography on silica gel, eluting with petroleum ether/ethyl ether mixtures.

400 Mg of the pure compound were obtained, m.p.

140-170°C.

EXAMPLE 2

a) Methyl 3α,7β-dicathyl-5β-cholanoate

2 Ml of ethyl chlorocarbonate were added dropwise to a solution of ursodeoxycholic acid methyl ester (2.9 g) in anhydrous dioxane (300 ml) and pyridine, at 0°C, under magnetic stirring. The reaction mixture was stirred at room temperature for 6 hours, then poured into water. The resulting solution was extracted with some portions of ethyl ether, and the combined organic extracts were washed with a 10% HCl solution, then with a saturated solution of NaCl, dried over anhydrous $MgSO_4$ and evaporated under vacuum. The residue was subjected to chromatography on silica gel, eluting with petroleum ether/ethyl ether. 2.2 Grams of the dicathylate derivative were obtained.

b) Methyl 3α,7β-dicathyl-23-ethyl-5β-cholanoate

4.7 Ml of n-butyl-lithium were added, during 20 min., to a solution of N-cyclohexyl-N-isopropylamine (1.4 ml) in anhydrous THF (65 ml); ursodeoxycholic acid methyl ester prepared in a) (1.2 g), dissolved in THF (15 ml) was added to the reaction mixture, under stirring and in nitrogen stream at -78°C. Thereafter ethyl iodide (3 ml) was added dropwise, and the mixture was stirred for 5 hours. The reaction mixture was acidified with 10% HCl, extracted with some portions of chloroform and the combined organic extracts were washed with water, dried over anhydrous $Na_2SO_4$ and evaporated under vacuum. By chromatography on silica gel, eluting with chloroform, 850 mg of the pure title compound were obtained.

I.R. ($CHCl_3$) = 1735 $cm^{-1}$

$^1$H-NMR ($CDCl_3$) $\delta$ = 0.64 (d, 3H, C-18, Me); 0.97 (s, 3H, C-19, Me); 1.30 (t, 6H, $OCH_2\underline{CH_3}$); 3.63 (s, 3H, OMe); 4.13 (q, 4H, $O\underline{CH_2}CH_3$); 4.53 (m, 2H, C-3 and C-7, $\underline{CH}OCO_2Et$).

c) <u>3α,7ß-Dihydroxy-23-ethyl-5ß-cholanoic acid</u>

0.8 Grams of methyl 3α,7ß-dicathyl-23-ethyl-5ß-cholanoate were dissolved in a 15% NaOH hydroalcoholic solution and the mixture was refluxed under magnetic stirring overnight. The reaction mixture was poured into water, acidified with concentrated HCl and extracted with some portions of chloroform. The combined organic fractions were washed with a saturated NaCl solution, dried over anhydrous $Na_2SO_4$ and evaporated under vacuum. The resulting residue was purified by chromatography on silica gel, to obtain 600 mg of the title acid.

P.f. 109-120°C.

I.R. ($CHCl_3$) 1710 $cm^{-1}$.

The compounds of the invention have a lower critic mycellar concentration (C.M.C.) than that of natural biliary acids, a longer lasting action and an increase of secretion per hour for the three biliary lipids, in a particularly selective way for the phospholipids and the biliary acids.

Therefore, thanks to their remarkable choleretic effect and of the positive effect on the biliary secretion, above all in comparison with analogous physiologic derivatives, the compounds of the invention are useful as choleretics, eupeptics, antidyspeptics, antidyslipidemics, in the treatment of calculosis and generally in all those pathologic conditions wherein a stimulus of the bile flow

and a qualitative and quantitative modification of the biliary composition are necessary.

An essential aspect of the present invention is therefore provided by pharmaceutical compositions containing as the active principle at least one of the compounds of formula I, in addition to the excipients of conventional use in pharmaceutical technique.

The compounds of the invention can be administered at doses ranging from 10 and 250 mg 2-3 times a day by the oral route, for instance in form of capsules, tablets, sugar-coated pills, monodose sachets or by local perfusion before or surgical operations, in form of solution of dispersable powders.

CLAIMS for the Contracting States:

BE, CH, DE, FR, GB, IT, LI, LU, NL, SE

1. Compounds of formula (I)

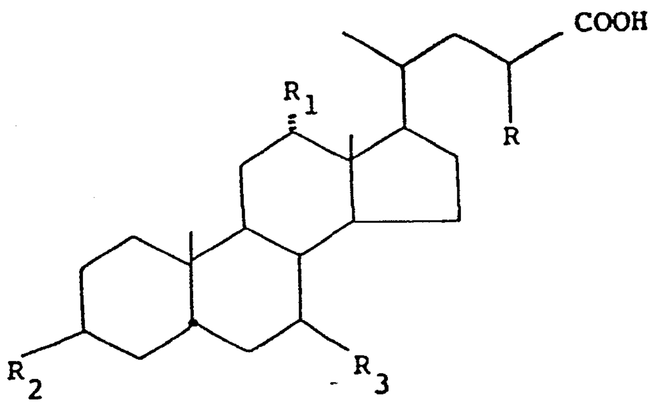

(I)

wherein:

R is hydrogen, methyl or ethyl;

$R_1$ is a $C_1$-$C_3$ alkoxy group and, when R is ethyl, $R_1$ may also be hydrogen or hydroxy;

$R_2$ and $R_3$, which may be the same or different, represent hydroxy groups both of α and ß configuration, or keto groups,

in all the stereisometric forms thereof, pharmaceutically acceptable salts and conjugates with glycine and taurine thereof.

2. Compounds of claim 1, wherein R is methyl and $R_1$ is methoxy.

3. Compounds of claim 1, wherein R is ethyl and $R_1$ is hydrogen.

4. Compounds of claim 1, wherein R is ethyl and $R_1$ is methoxy.

5. Compounds of claim 1, wherein R is hydrogen and $R_1$

is methoxy.

6. A compound selected in the group consisting of: 3α,7β-dihydroxy-23-ethyl-5β-cholanoic acid and 3α,7β-dihydroxy-12α-methoxy-23-methyl-5β-cholanoic acid.

7. A process for preparing compounds of claim 1, which process comprises reacting an alkyl halide, in the presence of a strong base, with an ester of the corresponding acid, having the hydroxy groups protected, and subsequently removing said protected groups and hydrolyzing the ester.

8. A process according to claim 7, wherein the strong base is a lithium alkyl.

9. A process according to claim 7, wherein the reaction is carried out at about -78°C, in tetrahydrofuran.

10. Pharmaceutical compositions containing as the active principle at least one compound of claims 1-6.

CLAIMS for AT:

1. Process for the preparation of compounds of formula I

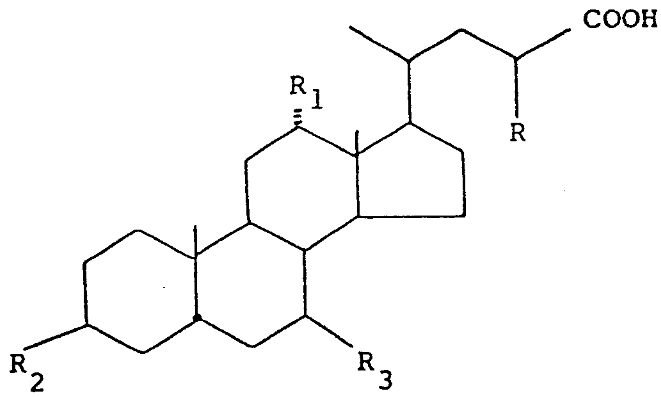

(I)

wherein:

R is hydrogen, methyl or ethyl;

$R_1$ is a $C_1$-$C_3$ alkoxy group and, when R is ethyl, $R_1$ may also be hydrogen or hydroxy;

$R_2$ and $R_3$, which may be the same or different, represent hydroxy groups both of α and ß configuration, or keto groups;

characterized by reacting an alkyl halide, in the presence of a strong base, with an ester of the corresponding acid, having the hydroxy groups protected, and subsequently removing said protected groups and hydrolyzing the ester.

2. A process according to claim 1 wherein the strong base is a lithium alkyl.

3. A process according to claim 1, wherein the reaction is carried out at about -78°C, in tetrahydrofuran.